# FASCICULE DE BREVET EUROPEEN

(11) **EP 3 449 007 B1**
(45) Date de publication et mention de la délivrance du brevet: **07.04.2021**
(21) Numéro de dépôt: 17724501.6
(22) Date de dépôt: 28.04.2017
(51) Int. Cl.: C12Q 1/04, C12M 1/12, C12N 1/20, C12Q 1/22, C12R 1/01, C12R 1/18

(54) **NOUVEAUX MICROORGANISMES TÉMOINS DE DÉCONTAMINATION**
NEUE DEKONTAMINATIONSERSATZMIKROORGANISMEN
NEW DECONTAMINATION SURROGATE MICROORGANISMS

(30) Priorité: 29.04.2016 FR 1653914
(43) Date de publication de la demande: 06.03.2019
(73) Titulaire: Novolyze, 21121 Daix (FR)
(72) Inventeur: ALVAREZ MARTIN, Pablo, 21560 Arc Sur Tille (FR); KHINOUCHE, Karim-Franck, 21000 Dijon (FR)
(74) Mandataire: Be IP
(86) Numéro de dépôt international: PCT/EP2017/060186
(87) Numéro de publication internationale: WO 2017/186907

(56) Documents cités:
- WO-A2-02/090904
- RODRIGUEZ O ET AL: "Surrogates for validation of electron beam irradiation of foods", INTERNATIONAL JOURNAL OF FOOD MICROBIOLOGY, ELSEVIER BV, NL, vol. 110, no. 2, 15 juillet 2006 (2006-07-15), pages 117-122, XP024956403, ISSN: 0168-1605, DOI: 10.1016/J.IJFOODMICRO.2006.01.041 [extrait le 2006-07-15]
- FUDGE JAMES ET AL: "The isolation and identification of Pantoea dispersa strain JFS as a non-pathogenic surrogate for Salmonella Typhimurium phage type 42 in flour", INTERNATIONAL JOURNAL OF FOOD MICROBIOLOGY, vol. 219, 16 février 2016 (2016-02-16), pages 1-6, XP029378817, ISSN: 0168-1605, DOI: 10.1016/J.IJFOODMICRO.2015.11.012 cité dans la demande
- ELENA ENACHE ET AL: "Development of a Dry Inoculation Method for Thermal Challenge Studies in Low-Moisture Foods by Using Talc as a Carrier for Salmonella and a Surrogate (Enterococcus faecium)", JOURNAL OF FOOD PROTECTION, vol. 78, no. 6, 1 June 2015 (2015-06-01), pages 1106-1112, XP055587261, US ISSN: 0362-028X, DOI: 10.4315/0362-028X.JFP-14-396

## Description

### DOMAINE DE L'INVENTION

La présente invention concerne la validation de procédés de décontamination, et en particulier de nouveaux organismes témoins, ainsi que des mélanges de ces microorganismes, employés pour valider les procédés de décontamination.

### ETAT DE LA TECHNIQUE

Les procédés de pasteurisation sont appliqués dans de multiples domaines, qu'il s'agisse de la stérilisation de matériel ou de la décontamination d'aliments, en particulier d'aliments secs. Ces procédés consistent en des étapes spécifiques de décontamination/stérilisation, ou bien sont le résultat concomitant d'une étape d'un procédé de traitement, comme par exemple une étape de cuisson d'un aliment, par exemple la torréfaction de produits d'origine végétale.

Les produits d'origine végétale, comme les amandes ou les épices sont souvent contaminés par des microorganismes pathogènes présents dans leur environnement de culture, de stockage et d'utilisation qui nécessite une étape de décontamination avant leur usage pour la consommation humaine. Souvent, cette décontamination est effectuée comme un traitement par la température de ces aliments, comme une cuisson, une torréfaction ou un séchage. Toutefois, certains pathogènes peuvent être résistants à certaines conditions de décontamination et il faut bien s'assurer, avant la mise en oeuvre du procédé que l'objectif de décontamination sera bien atteint.

Cette validation ne peut pas se faire avec un microorganisme pathogène à cause des risques de contamination. Pour ce faire, on emploie des microorganismes témoins dits « substituts », dont le comportement face aux conditions de traitement doit être proche de celui de l'organisme pathogène. De préférence, les substituts seront choisis pour être plus résistants aux conditions de traitement que les pathogènes, sans pour autant avoir un comportement trop éloigné de celui de ces pathogènes cibles.

Ces substituts sont généralement spécifiques d'un pathogène particulier dans un procédé de décontamination, comme par exemple *Enterococcus faecium* (ATCC 8459) recommandé pour la validation de procédés de pasteurisation des amandes susceptibles d'être contaminées par des salmonelles pathogènes.

Les substituts ne sont pas forcément les microorganismes plus proches philogénétiquement des pathogènes cibles, comme par exemple le genre *Citrobacterium,* genre plus roche d'un point de voie évolutive de *Salmonella,* n'est pas décrit comme substitut de ce pathogène. Ainsi, comme substitut de Salmonelle, certains ont employé *Geobacillus stearothermophilus* (ATCC 12980) pour la validation d'un procédé d'extrusion d'aliments pour animaux (Okelo et al., 2006 et 2008), *Enterococcus faecium* (NRRL B-2354) pour la pasteurisation des liquides (Annous & Kozempel, 1998), des amandes (ABC, 2007) ou l'extrusion des aliments (Bianchit, 2014), *Pantoea agglomerans* (SPS 2F-1) pour le grillage des amandes (ABC, 2007), *Pantoea dispersa* pour le traitement d'aliments frais par irradiation électronique ou « electron beam » (Fudge & al., 2016), *Pediococcus spp.* et *Pediococcus acidilactici* pour la préparation de bandes de bœuf séché (Borowski et al, 2009), la préparation de dinde séchée (Williams, 2010) ou l'extrusion d'aliments pour animaux (Ceylan and Bautista, 2015), et *Staphylococcus carnosus* (CS-299) pour la préparation de bœuf haché et de chair à saucisse (Vasan et al., 2014).

Comme substituts de *Clostridium botulinium,* certains ont employé *Clostridium sporogenes* (PA3679, 3676 et 3678) pour des aliments peu acides (Wallace et al. 2006)

Comme substituts de *Listeria monocytogenes,* certains ont employé *Listeria innocua* dans un procédé de pasteurisation de saucisses (Sommers et al., 2008) ou *Escherichia coli* K12 dans un procédé de stérilisation par irradiation des melons (Rodriguez et al., 2006).

Divers *E. coli* non pathogènes ont été décrits comme substituts de *E. coli* O157:H7 dans le traitement de jus (Gurtler, 2010) ou de bœuf (Garcia Hernandez et al., 2015).

Cependant que la souche *Enterococcus faecium* (NRRL B-2354) a été utilisée pour la validation de procédés thermiques pour plusieurs aliments à faible activité de l'eau, cette souche montre une thermorésistance beaucoup plus élevée qu'un grand nombre de pathogènes, notamment la Salmonelle.

De plus, hormis *Enterococcus faecium,* les souches identifiées de l'état de la technique le sont au niveau de tests de laboratoire, souvent sous formes de suspensions liquides extemporanées, peu versatiles quant aux supports employés et peu adaptées à un usage industriel à grande échelle qui nécessite la mise à disposition de grandes quantités de formes viables de substituts prêts à l'emploi sur de multiples supports.

Il reste un besoin de substituts mieux adaptés aux procédés de décontamination et aux pathogènes cibles, pouvant être employés seuls ou en mélanges, qui ont des comportements de résistance plus proche du ou des pathogènes cibles et apportent des informations plus pertinentes quant au procédé de décontamination de manière à valider des procédés plus économes en énergie et plus respectueux des propriétés structurelles et/ou organoleptiques des produits traités ; en particulier adaptés à un usage industriel. Les inventeurs ont mis en évidence plusieurs groupes de microorganismes non pathogènes répondant à ce besoin.

### EXPOSE DE L'INVENTION

La présente invention concerne un procédé de contrôle d'un procédé de décontamination dans lequel on met en œuvre le procédé de décontamination en présence d'au moins un microorganisme témoin, ou un mélange de microorganismes témoins, et on observe le comportement du ou des microorganismes témoins au cours dudit procédé de décontamination, caractérisé en ce que le microorganisme témoin est un microorganisme non pathogène choisi parmi les *Enterobacteriaceae* non pathogènes du genre *Enterobacter* choisies parmi les espèces *Enterobacter hormaechei* et *Enterobacter mori,* du genre *Erwinia* choisies parmi les espèces *Erwinia persicina* ou du genre Pantoea choisies parmi les espèces *Pantoea agglomerans* et *Pantoea calida,* et leurs mélanges.

La présente invention concerne aussi des microorganismes témoins susceptibles d'être employés comme substituts dans un procédé de contrôle d'un procédé de décontamination, choisis parmi *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055, *Pantoea calida* CNCM I-5056.

Elle concerne également un kit de contrôle d'un procédé de décontamination, comprenant au moins un microorganisme témoin selon l'invention et un support approprié pour son utilisation dans le procédé de décontamination.

### DESCRIPTION DETAILLEE DE L'INVENTION

La présente invention concerne un procédé de contrôle d'un procédé de décontamination dans lequel on met en œuvre le procédé de décontamination en présence d'au moins un microorganisme témoin, ou un mélange de microorganismes témoins, et on observe le comportement dudit du ou des un microorganismes témoins au cours dudit procédé de décontamination, caractérisé en ce que le microorganisme témoin est un microorganisme non pathogène choisi parmi les *Enterobacteriaceae* non pathogènes du genre *Enterobacter* choisies parmi les espèces *Enterobacter hormaechei* et *Enterobacter mori,* du genre *Erwinia* choisies parmi les espèces *Erwinia persicina* ou du genre Pantoea choisies parmi les espèces *Pantoea agglomerans* et *Pantoea calida,* et leurs mélanges.

Par « microorganisme » on entend un ensemble de plusieurs individus de microorganismes d'une même espèce. De préférence, les microorganismes sont appropriés pour un usage industriel, c'est à dire qu'ils peuvent être produits en grandes quantités par fermentation, jusqu'à au moins 10¹⁰ CFU/g, plus préférentiellement jusqu'à au moins 10¹¹ CFU/g.

On citera les bactéries choisies parmi les espèces *Enterobacter hormaechei, Enterobacter mori, Erwinia persicina, Pantoea agglomerans, Pantoea calida,* , non pathogènes, de préférence capables d'être produits de manière industrielle, plus particulièrement choisies parmi les espèces suivantes, déposées à la CNCM selon le Traité de Budapest : *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055, *Pantoea calida* CNCM I-5056.

Selon un premier mode de réalisation de l'invention, au moins un microorganisme témoin est choisi parmi les *Enterobacteriaceae* non pathogènes du genre *Pantoea,* du genre *Enterobacter* ou du genre *Erwinia* et leurs mélanges définis ci-dessus.

De manière avantageuse, on emploie un mélange d'au moins 2 microorganismes témoins (validation multiplexe), en particulier au moins 2 microorganismes témoins choisis parmi les *Enterobacteriaceae* non pathogènes du genre *Pantoea,* du genre *Enterobacter* ou du genre *Erwinia* et leurs mélanges définis ci-dessus. Selon un mode plus particulier de réalisation de l'invention, le mélange comprend au moins un microorganisme témoin choisi parmi les *Enterobacteriaceae* non pathogènes du genre *Pantoea* et au moins un microorganisme témoin choisi parmi les *Enterobacteriaceae* non pathogènes du genre *Enterobacter* ou du genre *Erwinia* telles que définies ci-dessus.

Ces microorganismes témoins non pathogènes ont pour avantage de montrer une résistance aux conditions de mise en œuvre de différents procédés de décontamination supérieure à celle d'au moins un organisme pathogène cible. Ces organismes pathogènes cibles sont des microorganismes responsables de contaminations, en particulier les bactéries pathogènes des genres *Salmonella, Escherichia, Bacillus, Listeria, Campylobacter, Cronobacter,* etc. Le procédé de décontamination qui fait l'objet du contrôle a pour but d'éliminer l'ensemble de ces pathogènes dans l'éventualité où ils seraient présents dans le produit traité.

De manière avantageuse, les microorganismes témoins ont une résistance thermique supérieure à *Salmonella* en conditions de faible aw et conditionnés sur une matrice inerte.

Par « faible aw », on entend de préférence une activité de l'eau inférieure à 0.85 (CAC/RCP 75-2015, Codex Alimentarius).

Par « matrice inerte » on entend de préférence un support approprié pour la conservation et l'emploi des microorganismes témoins, en particulier sous forme sèche. Le support est inerte, c'est à dire n'interagit pas avec le métabolisme des bactéries sous forme sèche permettant une conservation optimale dans le temps.

Dans le procédé de décontamination, le microorganisme témoin sera employé sous une forme appropriée, correspondant à la forme du pathogène ciblé susceptible d'être présent dans le produit à décontaminer, en particulier sous forme végétative et/ou forme végétative sèche.

Par « forme sèche » ou « forme végétative sèche » on entend bactéries sous végétative qui ont suivi un procédé de séchage permettant leur conservation pour une durée déterminée sans modification de ses caractéristiques de résistance.

Les microorganismes témoins produits par fermentation sont ensuite séchés pour leur conservation selon des techniques connues de l'homme du métier, comme la lyophilisation, l'atomisation ou le séchage.

Les procédés de décontamination comprennent généralement une ou plusieurs étapes de pasteurisation, séchage, extrusion, torréfaction, cuisson, stérilisation, autoclavage et traitements à la vapeur.

Ces procédés sont bien connus de l'homme du métier, notamment pasteurisation, séchage, extrusion, torréfaction, cuisson, stérilisation, autoclavage, traitements à la vapeur, lumière puisée, traitements à haute pression, ou irradiation, la stérilisation par les gaz (EtO, ppo, ozone) et par des désinfectants (eau de javel, acide peracétique...), en particulier pour le traitement de produits naturels ou manufacturés, comme les fruits à coques, herbes aromatiques, graines, épices, poudres alimentaires, aliments pour animaux de compagnie et bétail, céréales, etc.

Les substituts, et mélanges de substituts, selon l'invention pourront être employés, selon les aliments et procédés sélectionnés, pour valider les décontaminations de pathogènes tels que *Salmonella, Escherichia coli, Bacillus, Listeria, Campylobacter, Cronobacter sakazakii,* etc.

A cet effet, le microorganisme témoin sera employé avec un support approprié, bien connu de l'homme du métier, de préférence inerte, par exemple avec des cryoprotecteurs comme la maltodextrine et/ou poudre du lait et des supports solides comme le talc, la silice et/ou le charbon actif. Le support peut aussi comprendre un marqueur qui permet de retrouver facilement les produits contaminés (par exemple un colorant dans le visible ou fluorescent à une source UV/IR), en particulier tout marqueur permettant de distinguer les zones contaminées des autres (marquage magnétique, isotopique, chimique etc.).

L'usage d'un support approprié permet une standardisation en l'utilisation des microorganismes sur différents matrices en apportent à la fois une meilleure stabilité des microorganismes et évite d'avoir à valider la stabilité de chaque microorganisme témoin sur chaque support après inoculation. Il facilite la mise en œuvre du procédé selon l'invention.

L'invention concerne aussi une composition sèche comprenant un microorganisme témoin et un support approprié, tels que définis ci-dessus et ci-après.

La composition comprend avantageusement une teneur en microorganismes témoins d'au moins 10¹⁰ CFU/g de composition sèche.

La composition sèche est avantageusement une poudre qui a une activité de l'eau égale ou inférieure à 0,3.

Ces compositions sont préparées selon des méthodes connues de l'homme du métier en mélangeant selon les techniques usuelles, les microorganismes témoins sous une forme sèche avec le support, dans les proportions souhaitées. Selon un autre mode de réalisation, les microorganismes témoins sont mélangés au support approprié, le mélange étant ensuite séché pour sa conservation.

De manière générale, les microorganismes témoins avec leur support sont ajoutés aux produits à décontaminer en quantité appropriée pour permettre la vérification de l'efficacité du procédé de décontamination.

Les microorganismes et leur support pourront, le cas échéant, subir un traitement préalable à la décontamination, similaire à celui subi par le produit à décontaminer, c'est à dire qui va mimer les processus connus de contamination des produits. Par exemple, dans le cas de produits naturels qui sont broyés (épices notamment) il est possible de les broyer après ajout des microorganismes témoins sur leur support pour arriver à des poudres en recréant les conditions classiques de contamination des produits naturels.

Le procédé de contrôle selon l'invention peut être mis en œuvre avant toute mise en oeuvre d'un procédé de décontamination sur le produit à décontaminer, pour valider l'efficacité du procédé de décontamination (procédé de validation). Il peut aussi être mis en oeuvre pendant les opérations de décontaminations sur le produit à décontaminer, comme témoin de décontamination ou comme témoin de conformité de mise en oeuvre du procédé de décontamination (procédé de contrôle).

Le procédé selon l'invention, qu'il s'agisse d'un procédé de validation ou de contrôle peut être mis en oeuvre sous la responsabilité de celui qui réalise la décontamination ou encore sous celle d'un organisme de contrôle ou d'homologation.

Les microorganismes de contrôle seront avantageusement fournis sous forme de kit, avec leur support d'utilisation, et le cas échéant une notice d'emploi.

L'observation du comportement du microorganisme témoin consiste généralement à contrôler la présence d'individus viables, en cours de procédé de décontamination et/ou à son terme. Les méthodes employées sont connues de l'homme du métier : dénombrement des colonies sur gélose et/ou méthodes moléculaires comme la PCR et/ou qRT-PCR, ou des tests de détection de microorganismes comme les tests immunologique, par exemple les tests utilisant des technologies de SPR, comme ceux développés par la société PRESTODIAG, ou encore des tests de détection par phages.

La présente invention concerne également un microorganisme témoin susceptible d'être employé comme substitut dans un procédé de contrôle d'un procédé de décontamination, choisi parmi *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055, *Pantoea calida* CNCM I-5056. Elle concerne plus particulièrement ces microorganismes isolés, ou sous forme végétative et/ou forme végétative sèche. L'invention concerne également un mélange de microorganismes comprenant au moins 2 des espèces de microorganismes ci-dessus, dans toutes leurs combinaisons 2 à 2, jusqu'à un mélange comprenant les 10 espèces de microorganismes ci-dessus. L'invention concerne aussi une composition comprenant un microorganisme ci-dessus ou un mélange desdits microorganismes et un support approprié, en particulier un support inerte tel que définit plus haut.

Selon un mode préféré de réalisation de l'invention, le substitut est choisi parmi *Pantoea agglomerans* CNCM I-5054, *Pantoea agglomerans* CNCM I-5055, *Pantoea calida* CNCM I-5056 ou un mélange de substituts comprenant au moins un substitut choisi parmi *Pantoea agglomerans* CNCM I-5054, *Pantoea agglomerans* CNCM I-5055, *Pantoea calida* CNCM I-5056.

L'invention concerne aussi un kit de contrôle d'un procédé de décontamination, caractérisé qui comprend au moins un microorganisme ci-dessus et un support approprié tel que défini ci-dessus pour son utilisation dans le procédé de décontamination, et le cas échéant une notice d'utilisation.

L'invention concerne également l'utilisation d'au moins un microorganisme choisi parmi les *Enterobacteriaceae* non pathogènes du genre *Enterobacter,* du genre *Erwinia* ou du genre *Pantoea* tels que définis ci-dessus, ou un kit selon l'invention, pour le contrôle d'un procédé de décontamination, notamment pour une validation préalable ou un contrôle en cours d'exploitation.

### DESCRIPTION DES FIGURES

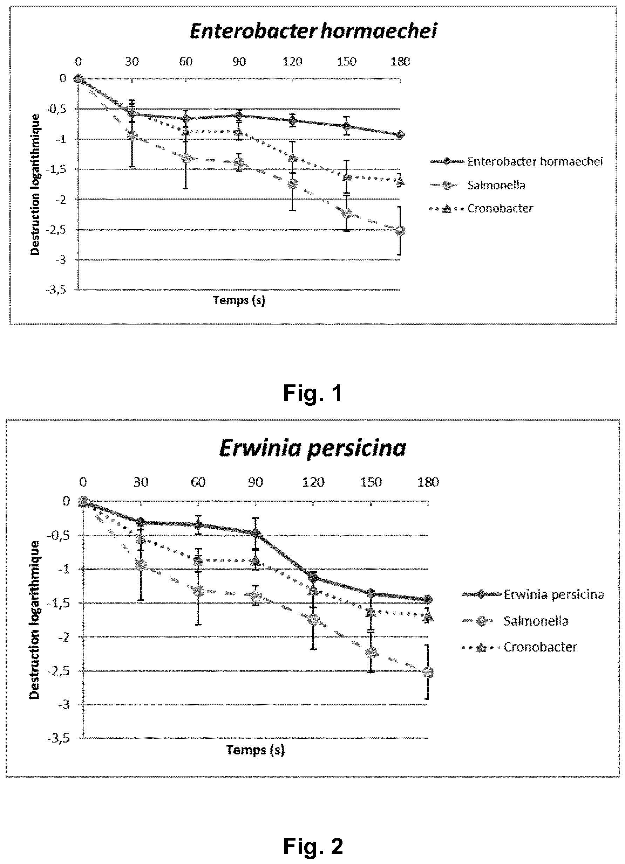

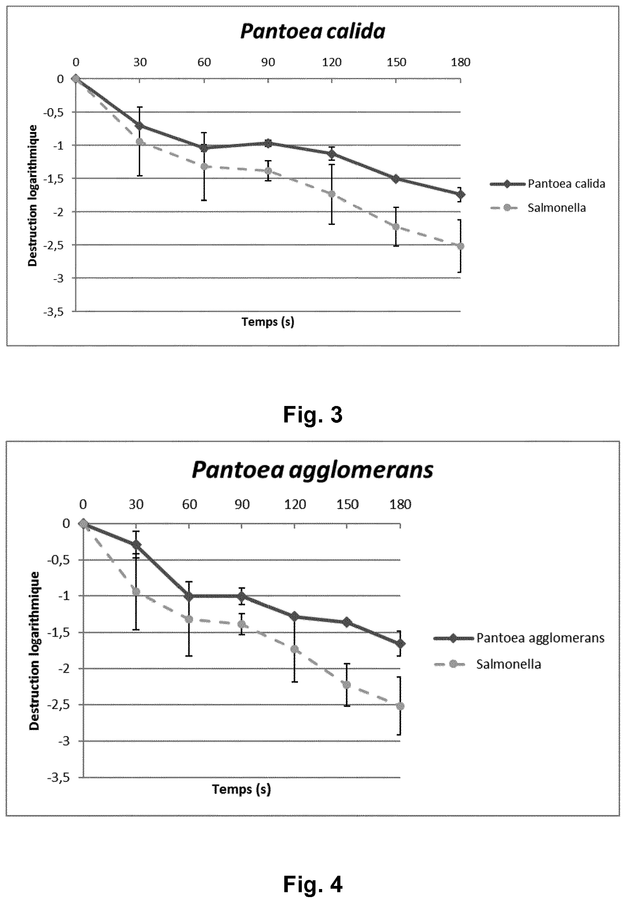

Les figures 1 à 4 représentent les courbes de résistances de différents microorganismes substituts en comparaison aux pathogènes *Salmonella* et *Cronobacter sakazakii.*
Les figures 5 à 7 représentent les dynamiques de destruction cellulaire de microorganismes témoins selon l'invention, de la souche référence E. *faecium* ATCC 8459 et de 4 sérotypes de *Salmonella* sur différents supports.

### EXEMPLES

### Exemple I. Isolation et sélection des microorganismes.

### 1. Isolement d'enterobacteriaceae environnementales

0.5 g d'échantillon de produits secs a été mis dans un Eppendorf de 1.5 mL et traité thermiquement dans un bain sec pendant 15 min à 95 °C. Après un refroidissement jusqu'à température ambiante, 1 mL de PBS concentré (aw= 0.950) a été ajouté avant d'effectuer un mélange pendant 30 s grâce à un vortex. Des dilutions successives au 10^{ième} ont été réalisées dans du PBS (aw = 0.995) avant étalement sur une gélose Violet Red Bile Glucose Agar (VRBG) à raison de 100 µL par boîte. Après incubation à 37°C pendant 24 - 48 h, les colonies ont été isolées sur des géloses Tryptic Soya Agar (TSA) et incubées de nouveaux à 37°C pendant 24 h.

### 2. Identification d'enterobacteriaceae environnementales

Des amplifications de l'ADNr 16S de chaque isolat ont été effectuées directement en repiquant les colonies dans le mix PCR. Les amorces utilisées étaient : 27F (5'-AGA GTT TGA TCM TGG CTC AG-3') et 1492R (5'-TAC GGH TAC CTT GTT ACG ACT T-3'). Pour réaliser les réactions PCR, le kit « Taq core kit » (Quiagen, France) a été utilisé. Brièvement, le mix PCR (50µL par réaction) pour une colonie est composé de 0.5 µM de chaque amorce, de 0.2 mM de mix dNTP, de 0.75 U de Taq polymérase et de 1 X de tampon contenant du MgCl₂. L'amplification a été vérifiée par électrophorèse sur gel d'agarose à 1% avant de séquencer les produits PCR par la méthode de Sanger. Les séquences obtenues ont été blastées dans la base de données du NCBI (BLASTn) et ainsi les isolats ont pu être identifiés. Douze isolats ont ensuite été sélectionnés.

### 3. Challenge thermique

### a. Conditions de culture des souches

Toutes les cultures ont été conservées dans du Tryptic Soy Broth (TSB, Sigma-Aldrich) avec 20% glycérol (Sigma-Aldrich) à -80°C. Pour la reprise des cultures, les bactéries ont été inoculées sur gélose TSA pendant 24 h à 37°C puis cinq colonies de chaque bactérie ont été repiquées dans 50 mL de TSB avant d'être incubées à 37°C pendant 8 h. Ces suspensions bactériennes sont ensuite diluées dans 50 mL de TSB neuf pour atteindre une densité optique (DO) de 0.01 à 600 nm. Des cultures en phase stationnaires de croissance sont ainsi obtenues après 20 h à 37°C.

### b. Inoculation de la poudre de lait

Pour chaque bactérie, les 50 mL de cultures sont centrifugées (3400 g, 10 min à 25°C) puis lavées deux fois dans 25 mL de PBS. Finalement, une dernière centrifugation est effectuée, le surnageant est retiré et les culots sont pesés. La poudre de lait (26% de matière grasse) est ajoutée sur chaque culot avec un ratio de 1:20 (m_{culot}:m_{poudre}) et le tout est homogénéisé à l'aide d'un mortier. Une poudre de lait inoculée est ainsi obtenue.

### c. Procédé de séchage

Pour la poudre de lait inoculée, des boîtes hermétiques, contenant des solutions saturées en sel permettant de contrôler l'activité de l'eau et donc l'humidité relative de l'atmosphère, sont utilisées. Le chlorure de lithium, le potassium acétate, le potassium carbonate et le bromure de sodium ont été utilisés pour obtenir une activité de l'eau de 0.11, 0.25, 0.44 and 0.58. Les atmosphères ainsi obtenues sont maintenues sous convection en utilisant un ventilateur (. Pour chaque souche, la poudre inoculée est étalée dans des boîtes de Petri (environ 5 g par boîte). Ces boîtes sont ensuite places sans couvercle dans les boîtes hermétiques pendant 16 h pour atteindre l'activité de l'eau d'équilibre. Tous les séchages ont été effectués à température ambiante.

### d. Traitement thermique

0,1 g de poudre de lait inoculée séchée est placé dans un tube de 0.2 mL et traité à différentes températures (85°C, 90°C, 95°C et 100°C) pendant un temps donné (0 s, 30 s, 60 s, 90s, 120 s, 150s and 180 s) grâce à un thermocycleur avant d'être refroidi à 4°C. Les échantillons sont réhydratés par addition de 1 mL de PBS avant agitation au vortex pendant 30 s. Un dénombrement des UFC a été réalisé après incubation sur TSA pendant 24 h à 37°C. Les résultats sont exprimés en log₁₀(N/N₀), où N représente les UFC après traitement and N₀ représente les UFC initiale de la poudre de lait avant traitement (t = 0 s).

| **Espèce** | **N° Dépôt** | **D-value** |
|---|---|---|
| *Enterococcus faecium* | ATCC 8459 | 23.69 |
| *Enterobacter hormaechei* | CNCM I-5058 | 4.36 |
| *Pantoea agglomerans* | CNCM I-5059 | 1.81 |
| *Enterobacter mori* | CNCM I-5060 | 3.95 |
| *Pantoea calida* | CNCM I-5061 | 2.03 |
| *Erwinia persicina* | CNCM I-5062 | 1.74 |
| *Erwinia persicina* | CNCM I-5063 | 1.96 |
| *Pantoea agglomerans* | CNCM I-5054 | 1.27 |
| *Pantoea agglomerans* | CNCM I-5055 | 1.33 |
| *Pantoea calida* | CNCM I-5056 | 1.14 |
| *Salmonella Typhimurium* | DSM 10506 | 1.21 |
| *Cronobacter sakazakii* | CIP 103183T | 1.13 |

### Exemple II. Validation d'un procédé de décontamination.

La technologie la plus communément utilisée dans le secteur de la décontamination, aussi bien en industries agroalimentaires que pharmaceutiques, reste les autoclaves. Il est ainsi possible de chauffer le produit dans une chambre, statique ou en mouvement, simplement par condensation de vapeur sur ce dernier. Le séchage du produit peut ensuite se faire par une combinaison de chauffage et de mise sous vide. Il existe des centaines de fabricants d'autoclaves dans le monde, dont un certain nombre travaille sur la pasteurisation des produits alimentaires secs.

Le cycle classique de pasteurisation de ces équipements se compose des étapes suivantes :
- Phase 1 : Elimination de l'air. Plusieurs cycles sont réalisés afin d'éliminer autant d'air que possible. Cette étape est nécessaire pour permettre à la vapeur de pénétrer à travers le produit.
- Phase 2 : Chauffage. La vapeur est injectée dans le but de chauffer le produit. L'enceinte de la chambre est aussi chauffée par des résistances électriques pour éviter tout phénomène de condensation.
- Phase 3 : Pasteurisation. Une fois que le produit a atteint une température cible, il y a un pallier de maintien à cette température. Le couple temps-température de traitement est défini en amont du travail de validation. Le couple temps-température est crucial pour l'efficacité du traitement.
- Phase 4 : Séchage. La vapeur est évacuée par un séchage sous vide.
- Phase 5 : Aération. La chambre est aérée par un flux d'air filtré à pression atmosphérique

Le produit est ensuite évacué de la chambre dans le sens de production, il ne peut croiser la matière non traitée. Selon le cycle choisi, sa température de fin de procédé varie de 30 à 50°C. Le produit n'est pas conditionné avant de n'être revenu à température ambiante car tout ensachage trop chaud pourrait provoquer un développement de germes.

La validation d'un procédé de décontamination in-situ comprend en général trois étapes principales :
- Phase préparatoire : évaluation du procédé, évaluation des risques, qualification du germe modèle, élaboration du protocole de validation *in-situ*
- Phase d'exécution : inoculation du produit à tester, exécution des « lots » de validation, récupération des échantillons
- Phase de synthèse : dénombrement des germes modèles, rédaction du rapport d'analyse et / ou rapport de validation

Lors de l'élaboration du protocole de validation sont définis, entre autres :
- La nécessité ou non de procéder à un pré-traitement du produit à tester (par exemple : irradiation)
- Le nombre de lots de validation ainsi que la durée de chaque lot de validation à réaliser
- La quantité de produit à inoculer (de 25g à >10t en fonction des procédés de décontamination et de la méthode de validation sélectionnée)
- Le niveau d'inoculation souhaité ainsi que la quantité de germe modèle à utiliser
- La méthode d'inoculation du produit avec le germe modèle (différentes possibilités existent, incluant l'inoculation en laboratoire, directement en usine, chez un prestataire de service...)
- La méthode d'échantillonnage en sortie de ligne de production (incluant entre autres le nombre et la taille des échantillons)
- La méthode de dénombrement du germe modèle (entre autres : milieu sélectif ou non)

Les réponses à ces différentes questions dépendent essentiellement de trois paramètres : type de procédé à valider, pathogène cible, produit à inoculer.

Ainsi, le « kit » de validation fourni peut varier en particulier en :
- Niveau de mélange du germe modèle avec le produit à tester (le germe modèle peut-être fourni sous forme concentrée à inoculer ou sous forme pré-mélangée avec le produit)
- Niveau de concentration en germe modèle
- Quantité fournie (de plusieurs kgs / dizaines de kgs pour la forme concentrée à plusieurs tonnes pour le version pré-mélangée)

### Exemple III. Production de microorganismes témoins par fermentation

### Pré-culture

La pré-culture du microorganisme substitut doit être lancée entre 16h et 24h avant la fermentation. L'erlenmeyer contenant le milieu de culture est ensemencé avec microorganisme substitut selon un ratio 1 :5. La pré-culture est incubée à 37°C sous des conditions d'agitation de 150 rpm .

### Conduite de la fermentation

La culture débute lorsque toute la pré-culture a été inoculée dans le fermenteur. Les conditions d'agitation, d'aération et d'ajout de substrats sont les suivantes :
- pH maintenu par une base durant toute la culture
- Température maintenue à 37°C par la double enveloppe et/ou manteau chauffant, et durant toute la culture
- Saturation en oxygène du milieu initial avant inoculation (pO2 > 90%)
- Agitation (rpm) : 200-500.
- Aération (L/min) : 1-3.

A la fin de la culture le milieu de culture du fermenteur est transféré dans des bouteilles stériles afin de récupérer toute la biomasse.

### Récupération de la biomasse et mise en forme sèche

La biomasse est récupérée par centrifugation ou par une autre technique comme l'ultrafiltration que nous permettre de séparer les cellules du milieu de culture.

Le cryoprotectant, une fois stérilisé est ajoutée selon un rapport volumique 1 :1 sur la biomasse et l'ensemble est congelé à -80°C pendant au moins 24h en vue d'une potentielle lyophilisation.

### Exemple IV. Utilisation de microorganismes témoins sur différents supports secs

L'objectif de ces exemples est de montrer la cinétique de destruction thermique de différents microorganisme témoins et de les comparer à celle de Salmonella dans différents produits à faible activité de l'eau.

### Micro-organismes testés

Quatre sérotypes différents de Salmonella (Senftenberg, Entendis, Typhimurium et Mbandaka), inoculées de façon individuelle ou sous forme de cocktail, sont utilisées comme des souches contrôle pour la comparaison avec les microorganismes modèles testés.

La résistance thermique de deux microorganismes témoins différents est testée :
- *Enterobacter hormaechei* CNCM I-5058, préparation sèche.
- *Pantoea agglomerans* CNCM I-5055, préparation sèche.

La souche *Enterococcus faecium* (ATCC® 8459™) a été utilisée comme souche de référence car elle est largement utilisée comme traceur biologique en validation des procédés de traitements des produits alimentaires secs

### Protocoles d'inoculation

Deux méthodes différentes sont utilisées pour inoculer les différents produits :
- Inoculation liquide des pathogènes : des cultures en bouillon des quatre sérotypes de Salmonella préparées la veille sont utilisées indépendamment pour inoculer les produits produits (poudre de paprika, poudre de lait et noix de macadamia). Après l'inoculation le produit est placée pendant sous une enceinte de sécurité biologique de type II afin d'équilibrer son activité de l'eau.
- Inoculation sèche des microorganismes témoins : le différents produits (poudre de paprika, poudre de lait et noix de macadamia) ont été inoculées indépendamment avec les microorganismes substituts sous forme sèche suite à un procédé de production par fermentation et une stabilisation par lyophilisation. Aucun temps de repos n'est requis après l'inoculation avec les microorganismes modèle sous forme sèche, entraîne une modification très faible des propriétés du produit (aw, pourcentage d'humidité...), ce qui permet une utilisation plus rapide des matrices inoculées.

### Résultats

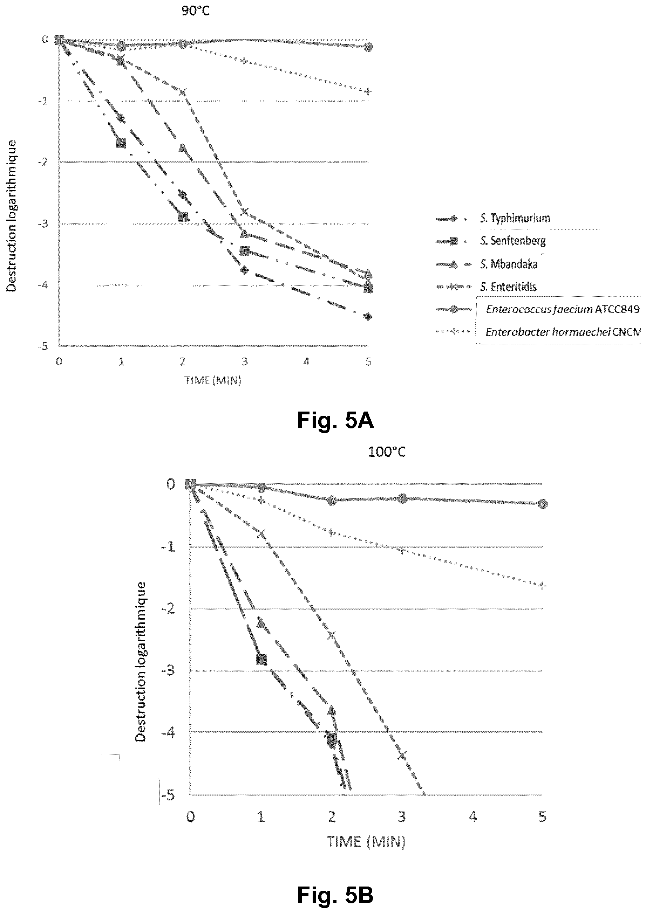

La dynamique de destruction cellulaire à 90°C et 100°C de la souche *E. hormaechei* CNCM I-5058, la souche référence *E. faecium* ATCC 8459 et les 4 serotypes de *Salmonella* sur la poudre de paprika est représentée sur les figures 5A (traitement à 90 °C) et 5B (traitement à 100°C). Les résultats montrent dans tous les cas un comportement du microorganisme modèle *E. hormaechei* CNCM I-5058 toujours plus proche de tous les serotypes de *Salmonella,* confirment de cette façon son caractère de germe substitut bien adapté au pathogène cible sur le produit en question.

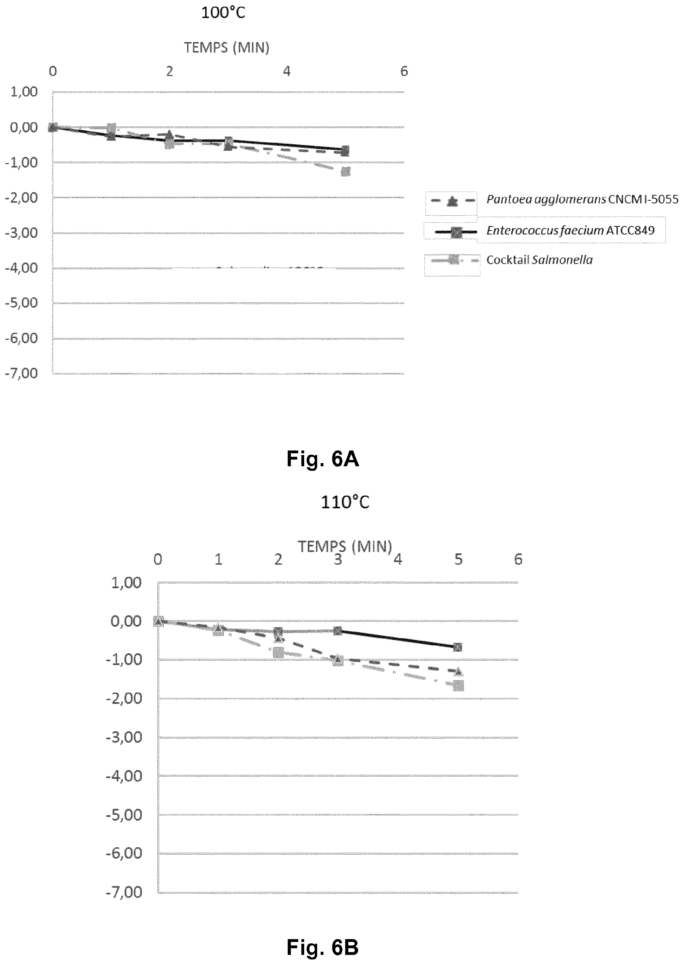

La dynamique de destruction cellulaire à 100°C et 110°C de la souche *P. agglomerans* CNCM I-5055, la souche référence *E. faecium* ATCC 8459 et les 4 serotypes de *Salmonella* sous forme de cocktail, sur les noix de macadamia est représentée sur les figures 6A (traitement à 100 °C) et 6B (traitement à 110°C). Les résultats montrent un comportement du microorganisme modèle *P. agglomerans* CNCM I-5055 plus proche de tous les serotypes de *Salmonella,* confirment de cette façon son caractère de germe substitut bien adapté au pathogène cible sur le produit en question.

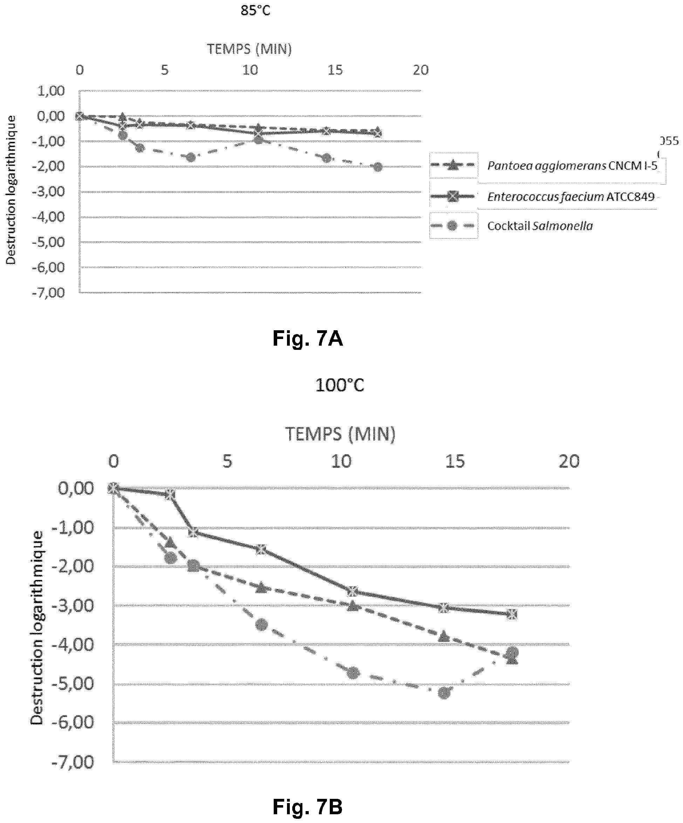

La dynamique de destruction cellulaire à 85°C et 100°C de la souche *P. agglomerans* CNCM I-5055, la souche référence *E. faecium* ATCC 8459 et les 4 serotypes de *Salmonella* sous forme de cocktail, sur la poudre de lait écrémé est représentée sur les figures 7A (traitement à 85 °C) et 7B (traitement à 100°C). Les résultats montrent un comportement du microorganisme modèle *P. agglomerans* CNCM I-5055 plus proche de tous les serotypes de *Salmonella,* confirment de cette façon son caractère de germe substitut bien adapté au pathogène cible sur le produit en question.

### RÉFÉRENCES

- Annous, 1998Annous BA, Kozempel MF. 1998. Influence of growth medium on thermal résistance of Pediococcus sp. NRRL B-2354 (formerly Micrococcus freudenreichii) in liquid foods. J Food Prot. 61(5):578-81.
- Bianchini & al., Use of Enterococcus faecium as a Surrogate for Salmonella enterica during Extrusion of a Balanced Carbohydrate-Protein Meal. J. Food Prot., Vol. 77, No. 1
- Borowski, A.G., S.C. Ingham, and B.H. Ingham, 2009. Validation of ground-and formed beef jerky processes using commercial lactic acid bacteria starter cultures as pathogen surrogates. Journal of Food Protection 72: 1234-1247
- Enache & al., Development of a Dry Inoculation Method for Thermal Challenge Studies in Low-Moisture Foods by Using Talc as a Carrier for Salmonella and a Surrogate (Enterococcus faecium). Journal of Food Protection, 2015. 78: 1106-1112
- Erdogan & al., Evaluating Pediococcus acidilactici and Enterococcus faecium NRRL B-2354 as Thermal Surrogate Microorganisms for Salmonella for In-Plant Validation Studies of Low-Moisture Pet Food Products. Journal of Food Protection, Vol. 78, No. 5, 2015, Pages 934-939.
- Fudge & al., The Isolation and Identification of Pantoea dispersa strain JFS as a Non-Pathogenic Surrogate for Salmonella Typhimurium Phage Type 42 in Flour, International Journal of Food Microbiology 219 (2016) 1-6
- Garcia Hernandez et al., 2015Garcia-Hernandez R, McMullen L, Gänzle MG. 2015. Development and validation of a surrogate strain cocktail to evaluate bactericidal effects of pressure on verotoxigenic Escherichia coli. Int J Food Microbiol. 205:16-22.
- Guidelines for Using Enterococcus faecium NRRL B-2354 as a Surrogate Microorganism in Almond Process Validation. Almond Board of California Guideline, October 2007 (ABC, 2007).
- Gurtler & al., Selection of surrogate bacteria in place of E. coli O157:H7 and Salmonella Typhimurium for pulsed electric field treatment of orange juice. International Journal of Food Microbiology 139 (2010) 1-8
- Kopit . B. Kim, R. J. Siezen, L. J. Harris, and M. Marco. & al., Safety of the Surrogate Microorganism Enterococcus faecium NRRL B-2354 for Use in Thermal Process Validation, Appl. Environ. Microbiol. 2014, 80(6):1899. DOI:10.1128/AEM.03859-13.
- Niebuhr & al., Evaluation of non-pathogenic surrogate bacteria as process validation indicators for Salmonella enteric for selected antimicrobial treatments, cold storage and fermentation in meat, J Food Prot. 2008 Apr; 71(4):714-8.
- Okelo, P. O., D.D. Wagner, L.E. Carr, F.W. Wheaton, L.W. Douglass, S.W. Joseph. 2006. Optimization of extrusion conditions for elimination of mesophilic bacteria during thermal processing of animal feed mash. Animal Feed Science and Technology 129:116-137.
- Okelo, P. O., S. W. Joseph, D. D. Wagner, F. W. Wheaton, L. W. Douglass, and L. E. Carr, 2008. Improvements in Réduction of Feed Contamination: An Alternative Monitor of Bacterial Killing During Feed Extrusion. Journal Applied Poultry Research 17: 219-228.
- Rodriguez et al., Surrogates for validation of electron beam irradiation of foods, International Journal of FDood Microbiology, 110 (2006) 117-122
- Sommers CH, Geveke DJ and, Fan X. Inactivation of Listeria Innocua on Frankfurters That Contain Potassium Lactate and Sodium Diacetate by Flash Pasteurization. 2008. J Food Sci 73 (2), M72-M74. 3 2008
- Vasan et al., 2014Vasan, A., R. Geier, S. C. Ingham, and B. H. Ingham. 2014. Thermal tolerance of O157 and non-O157 Shiga toxigenic strains of Escherichia coli, Salmonella, and potential pathogen surrogates, in frankfurter batter and ground beef of varying fat levels. Journal of Food Protection. 77:1501-11.
- Larson and Johnson. 2003 Wallace M, Larson K, Wolf I, Thompson D and Zottola E. Thermal inactivation of Clostridium sporogenes PA 3679 and Bacillus stearothermophilus 1518 in low-acid home-canned foods.. 2006 Journal of Food Science 43(6):1738 - 1740.
- Williams, 2010Williams, P., W. M. Leong, B. H. Ingham, S. C. Ingham, 2010. Lethality of Small-Scale Commercial Dehydrator and Smokehouse/Oven Drying Processes Against Escherichia coli O157:H7-, Salmonella spp.-, Listeria monocytogenes-, and Staphylococcus aureus-inoculated Turkey Jerky and the Ability of a Lactic Acid Bacterium to Serve as a Pathogen Surrogate. Poster presented at the annual meeting of the Institute of Food Technologists. Chicago, IL. July 2010.

## Revendications

1. Procédé de contrôle d'un procédé de décontamination dans lequel on met en œuvre le procédé de décontamination en présence d'au moins un microorganisme témoin et on observe le comportement dudit au moins un microorganisme témoin au cours dudit procédé de décontamination, **caractérisé en ce que** le au moins un microorganisme témoin est choisi parmi les *Enterobacteriaceae* non pathogènes du genre Pantoea choisies parmi les espèces *Pantoea agglomerans* et *Pantoea calida,* du genre *Enterobacter* choisies parmi les espèces *Enterobacter hormaechei* et *Enterobacter mori,* ou du genre *Erwinia* choisies parmi les espèces *Erwinia persicina* et leurs mélanges.

2. Procédé selon la revendication 1, **caractérisé en ce que** les microorganismes témoins sont employés sous forme végétative sèche.

3. Procédé selon l'une des revendications 1 ou 2, **caractérisé en ce que** les microorganismes témoins sont employés secs sur un support inerte.

4. Procédé de contrôle selon l'une des revendications 1 à 3, **caractérisé en ce que** les microorganismes témoins choisis parmi les *Enterobacteriaceae* non pathogènes sont choisis parmi *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055, *Pantoea calida* CNCM I-5056, et leurs mélanges.

5. Procédé de contrôle selon l'une des revendication 1 à 4, **caractérisé en ce que** le au moins un microorganisme témoin est choisi parmi *Pantoea agglomerans* CNCM I-5054, *Pantoea agglomerans* CNCM I-5055, *Pantoea calida* CNCM I-5056 et leurs mélanges.

6. Procédé selon la revendication 1, caractérisé en ce l'on emploie un mélange d'au moins 2 microorganismes témoins.

7. Procédé selon l'une des revendications 1 à 6, **caractérisé en ce que** le procédé de décontamination comprend une ou plusieurs étapes de pasteurisation, séchage, extrusion, torréfaction, cuisson, stérilisation, autoclavage et traitements à la vapeur.

8. Procédé de contrôle selon l'une des revendications 1 à 7, **caractérisé en ce que** le procédé de décontamination contrôlé a pour objet l'élimination d'un ou plusieurs microorganismes pathogènes cibles, choisis parmi *Salmonella, Escherichia coli, Bacillus, Listeria, Campylobacter, Cronobacter sakazakii.*

9. Microorganismes témoins susceptibles d'être employés comme substituts dans un procédé de contrôle d'un procédé de décontamination, **caractérisés en ce qu'**ils sont choisis parmi *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055 et *Pantoea calida* CNCM I-5056.

10. Mélange de microorganismes témoins, **caractérisé en ce qu'**il comprend au moins 2 espèces de microorganismes selon la revendication 9.

11. Composition sèche **caractérisée en ce qu'**elle comprend au moins un microorganisme témoin selon la revendication 9 et un support inerte.

12. Composition selon la revendication 11, **caractérisée en ce qu'**elle comprend une teneur en microorganismes témoins d'au moins 10¹⁰ CFU/g de composition sèche.

13. Composition sèche selon l'une des revendications 11 ou 12, **caractérisée en ce que** le au moins un microorganisme témoin est choisi parmi *Pantoea agglomerans* CNCM I-5054, *Pantoea agglomerans* CNCM I-5055 et *Pantoea calida* CNCM I-5056.

14. Kit de contrôle d'un procédé de décontamination, **caractérisé en ce qu'**il comprend au moins un microorganisme selon la revendication 9 et un support approprié pour son utilisation dans le procédé de décontamination.

15. Utilisation d'au moins un microorganisme selon la revendication 9 ou d'un mélange selon la revendication 10 ou d'une composition sèche selon l'une des revendications 11 à 13 ou d'un kit de contrôle selon la revendication 14 pour le contrôle d'un procédé de décontamination.

## Patentansprüche

1. Verfahren zur Kontrolle eines Dekontaminierungsverfahrens, wobei das Dekontaminierungsverfahren in Anwesenheit von mindestens einem Kontrollmikroorganismus durchgeführt wird und das Verhalten des mindestens einen Kontrollmikroorganismus während dieses Dekontaminierungsverfahrens beobachtet wird, **dadurch gekennzeichnet, dass** der mindestens eine Kontrollmikroorganismus ausgewählt ist aus nicht-pathogenen *Enterobacteriaceae* der Gattung *Pantoea,* ausgewählt aus den Spezies *Pantoea agglomerans* und *Pantoea calida,* der Gattung *Enterobacter,* ausgewählt aus den Spezies *Enterobacter hormaechei* und *Enterobacter mori,* oder der Gattung *Erwinia,* ausgewählt aus den Spezies *Erwinia persicina* und Mischungen davon.

2. Verfahren nach Anspruch 1, **dadurch gekennzeichnet, dass** die Kontrollmikroorganismen in trockener vegetativer Form verwendet werden.

3. Verfahren nach einem der Ansprüche 1 oder 2, **dadurch gekennzeichnet, dass** die Kontrollmikroorganismen trocken auf einem inerten Träger verwendet werden.

4. Kontrollverfahren nach einer der Ansprüche 1 bis 3, **dadurch gekennzeichnet, dass** die aus den nicht-pathogenen *Enterobacteriaceae* ausgewählten Kontrollmikroorganismen ausgewählt sind aus *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055, *Pantoea calida* CNCM I-5056 und Mischungen davon.

5. Kontrollverfahren nach einem der Ansprüche 1 bis 4, **dadurch gekennzeichnet, dass** der mindestens eine Kontrollmikroorganismus ausgewählt ist aus *Pantoea agglomerans* CNCM I-5054, *Pantoea agglomerans* CNCM I-5055, *Pantoea calida* CNCM I-5056 und Mischungen davon.

6. Verfahren nach Anspruch 1, **dadurch gekennzeichnet, dass** eine Mischung von mindestens zwei Kontrollmikroorganismen verwendet wird.

7. Verfahren nach einem der Ansprüche 1 bis 6, **dadurch gekennzeichnet, dass** das Dekontaminierungsverfahren eine oder mehrere Schritte der Pasteurisierung, der Trocknung, der Extrusion, der Röstung, des Kochens, der Sterilisation, der Autoklavierung und der Dampfbehandlung umfasst.

8. Kontrollverfahren nach einem der Ansprüche 1 bis 7, **dadurch gekennzeichnet, dass** das kontrollierte Dekontaminierungsverfahren dazu bestimmt ist, ein oder mehrere pathogene Zielmikroorganismen zu beseitigen, ausgewählt aus *Salmonella, Escherichia coli, Bacillus, Listeria, Campylobacter, Cronobacter sakazakii.*

9. Kontrollmikroorganismen, die als Substitute in einem Verfahren zur Kontrolle eines Dekontaminierungsverfahrens verwendet werden können, **dadurch gekennzeichnet, dass** sie ausgewählt sind aus *Enterobacter hormaechei* CNCM I-5058, *Pantoea agglomerans* CNCM I-5059, *Enterobacter mori* CNCM I-5060, *Pantoea calida* CNCM I-5061, *Erwinia persicina* CNCM I-5062, *Erwinia persicina* CNCM I-5063, *Pantoea agglomerans* CNCM I-5054, *Pantoea agglomerans* CNCM I-5055 und *Pantoea calida* CNCM I-5056.

10. Mischung von Kontrollmikroorganismen, **dadurch gekennzeichnet, dass** sie mindestens zwei Spezies von Mikroorganismen nach Anspruch 9 umfasst.

11. Trockene Zusammensetzung, **dadurch gekennzeichnet, dass** sie mindestens einen Kontrollmikroorganismus nach Anspruch 9 und einen inerten Träger umfasst.

12. Zusammensetzung nach Anspruch 11, **dadurch gekennzeichnet, dass** sie einen Gehalt an Kontrollmikroorganismen von mindestens 10¹⁰ KBE/g trockener Zusammensetzung aufweist.

13. Trockene Zusammensetzung nach einem der Ansprüche 11 oder 12, **dadurch gekennzeichnet, dass** der mindestens eine Kontrollmikroorganismus ausgewählt ist aus *Pantoea agglomerans* CNCM I-5054, *Pantoea agglomerans* CNCM I-5055 und *Pantoea calida* CNCM I-5056.

14. Kontroll-Kit für ein Dekontaminierungsverfahren, **dadurch gekennzeichnet, dass** es mindestens einen Mikroorganismus nach Anspruch 9 und einen für die Verwendung in dem Dekontaminierungsverfahren geeigneten Träger umfasst.

15. Verwendung mindestens eines Mikroorganismus nach Anspruch 9 oder einer Mischung nach Anspruch 10 oder einer trockenen Zusammensetzung nach einem der Ansprüche 11 bis 13 oder eines Kontroll-Kits nach Anspruch 14 für die Kontrolle eines Dekontaminierungsverfahrens.

## Claims

1. A process for monitoring a decontamination process wherein the decontamination process is implemented in the presence of at least one indicator microorganism and the behavior of said at least one indicator microorganism is observed during said decontamination process, **characterized in that** the at least one indicator microorganism is selected from non-pathogenic Enterobacteriaceae of the genus *Pantoea* selected from the species *Pantoea agglomerans* and *Pantoea calida,* of the genus *Enterobacter* selected from the species *Enterobacter hormaechei* and *Enterobacter mori,* or of the genus *Erwinia* selected from the species *Erwinia persicina* and mixtures thereof.

2. The process according to claim 1, **characterized in that** the indicator microorganisms are used in dry vegetative form.

3. The process according to any one of claims 1 or 2, **characterized in that** the indicator microorganisms are used dry on an inert carrier.

4. The monitoring process according to any one of claims 1 to 3, **characterized in that** the indicator microorganisms selected from non-pathogenic Enterobacteriaceae are selected from *Enterobacter hormaechei* CNCM 1-5058, *Pantoea agglomerans* CNCM 1-5059, *Enterobacter mori* CNCM 1-5060, *Pantoea calida* CNCM 1-5061, *Erwinia persicina* CNCM 1-5062, *Erwinia persicina* CNCM 1-5063, *Pantoea agglomerans* CNCM 1-5054, *Pantoea agglomerans* CNCM 1-5055, *Pantoea calida* CNCM 1-5056, and mixtures thereof.

5. The monitoring process according to any one of claims 1 to 4, **characterized in that** the at least one indicator microorganism is selected from *Pantoea agglomerans* CNCM 1-5054, *Pantoea agglomerans* CNCM 1-5055, *Pantoea calida* CNCM 1-5056, and mixtures thereof.

6. The process according to claim 1, **characterized in that** a mixture of at least 2 indicator microorganisms is used.

7. The process according to any one of claims 1 to 6, **characterized in that** the decontamination process comprises one or more steps of pasteurization, drying, extrusion, roasting, cooking, sterilization, autoclaving and steam treatments.

8. The monitoring process according to any one of claims 1 to 7, **characterized in that** the monitored decontamination process is aimed at removing one or more target pathogenic microorganisms selected from *Salmonella, Escherichia coli, Bacillus, Listeria, Campylobacter, Cronobacter sakazakii.*

9. Indicator microorganisms capable of being used as surrogates in a process for monitoring a decontamination process, **characterized in that** they are selected from *Enterobacter hormaechei* CNCM 1-5058, *Pantoea agglomerans* CNCM 1-5059, *Enterobacter mori* CNCM 1-5060, *Pantoea calida* CNCM I-5061, *Erwinia persicina* CNCM 1-5062, *Erwinia persicina* CNCM I-5063, *Pantoea agglomerans* CNCM 1-5054, *Pantoea agglomerans* CNCM 1-5055 and *Pantoea calida* CNCM 1-5056.

10. A mixture of indicator microorganisms, **characterized in that** it comprises at least 2 species of microorganisms according to claim 9.

11. A dry composition **characterized in that** it comprises at least one indicator microorganism according to claim 9 and an inert carrier.

12. The composition according to claim 11, **characterized in that** it comprises an indicator microorganism content of at least 10¹⁰ CFU/g dry composition.

13. The dry composition according to any one of claims 11 or 12, **characterized in that** the at least one indicator microorganism is selected from *Pantoea agglomerans* CNCM 1-5054, *Pantoea agglomerans* CNCM 1-5055 and *Pantoea calida* CNCM 1-5056.

14. A kit for monitoring a decontamination process, **characterized in that** it comprises at least one microorganism according to claim 9 and a carrier suitable for its use in the decontamination process.

15. Use of at least one microorganism according to claim 9 or of a mixture according to claim 10 or of a dry composition according to any one of claims 11 to 13 or of a monitoring kit according to claim 14 for monitoring a decontamination process.
